# EUROPÄISCHE PATENTANMELDUNG

(11) **EP 3 967 718 A1**
(43) Veröffentlichungstag der Anmeldung: **16.03.2022**
(21) Anmeldenummer: 20195677.8
(22) Anmeldetag: 11.09.2020
(51) Int. Cl.: C08G 18/24, C08G 18/48, C08G 18/67, C08G 18/75, C08G 18/81, C07C 7/00, C07C 7/04

(54) **VERFAHREN ZUR HERSTELLUNG ETHYLENISCH UNGESÄTTIGTER URETHANGRUPPEN-HALTIGER VERBINDUNGEN**

(71) Anmelder: Evonik Operations GmbH, 45128 Essen (DE)
(72) Erfinder: SPYROU, Emmanouil, 46514 Schermbeck (DE); LOESCH, Holger, 44627 Herne (DE); KREISCHER, Susanne, 45701 Herten (DE); DIESVELD, Andrea, 48712 Gescher (DE); THESING, Andrea, 48683 Ahaus (DE)
(74) Vertreter: Evonik Patent Association

(57) **Zusammenfassung**

Die vorliegende Erfindung betrifft ein Verfahren zur Herstellung mindestens einer ethylenisch ungesättigten Urethangruppen-haltigen Verbindung aus
i) Diisocyanat,
ii) mindestens einer Verbindung mit mindestens einer Hydroxygruppe und mindestens einer ethylenisch ungesättigten, polymerisierbaren Gruppe
iii) und optional mindestens einer hydroxygruppenhaltigen Verbindung, bei dem
1. Diisocyanat unmittelbar vor der Reaktion einer Peroxidgehaltbestimmung unterzogen wird,
2.
a) im Falle, dass der ermittelte Peroxidgehalt größer oder gleich 20 mmol/kg beträgt, das Diisocyanat einer destillativen Aufreinigung unterzogen wird, bis der ermittelte Peroxidgehalt kleiner als 20 mmol/kg ist,
oder
b) im Falle, dass der ermittelte Peroxidgehalt kleiner 20 mmol/kg beträgt, keine destillative Aufreinigung erfolgt, und

3. anschließend i) destilliertes Diisocyanat zu a) und/oder unbehandeltes Diisocyanat zu b) mit ii) mindestens einer Verbindung mit mindestens einer Hydroxygruppe und mindestens einer ethylenisch ungesättigten, polymerisierbaren Gruppe und optional ii) mindestens einer hydroxygruppenhaltigen Verbindung zu einer ethylenisch ungesättigten Urethangruppen-haltigen Verbindung umgesetzt wird.

## Beschreibung

Die vorliegende Erfindung betrifft ein Verfahren zur Herstellung mindestens einer ethylenisch ungesättigten Urethangruppen-haltigen Verbindung aus Diisocyanat und mindestens einer hydroxygruppenhaltigen Verbindung mit ethylenisch ungesättigten, polymerisierbaren Gruppen.

Urethangruppen-haltige Verbindungen mit ethylenisch ungesättigten Verbindungen spielen eine wichtige Rolle in strahlungshärtenden Beschichtungszusammensetzungen.

So offenbart z.B. US 4,260,703 A Esterdiol-Urethan-Acrylate, die für sich genommen oder zusammen mit anderen Bestandteilen wie Lösemitteln, reaktiven Oligomeren und Monomeren, Vernetzern, Füllstoffen oder anderen Additiven in Beschichtungszusammensetzungen verwendet werden können.

WO 2017/151387 A1 offenbart ein Verfahren zur Herstellung einer härtbaren Harzzusammensetzung, bei dem ein Isocyanat, ein Polyolgemisch und ein Verkappungsmittel, bei dem es sich um ein Hydroxyalkylacrylat oder -methacrylat handeln kann, umgesetzt werden. Die resultierenden Harzzusammensetzungen können zur Herstellung von Verbundstoffen, Beschichtungen, Klebstoffen, Druckfarben, Verkapselungen und Gussstücken eingesetzt werden.

Bislang wiesen die hergestellten ethylenisch ungesättigten Urethangruppen-haltigen Verbindungen oder ihre Vorstufen den Nachteil auf, zu einer unerwünschten vorzeitigen Polymerisation (Gelierung) zu neigen. Es bestand somit ein Interesse, diese vorzeitige Polymerisation zu vermeiden.

Überraschenderweise wurde festgestellt, dass insbesondere in älteren Chargen ethylenisch ungesättigter Urethangruppen-haltigen Verbindungen Peroxidverunreinigungen vorliegen, die zu der erwähnten Gelierung führen. Weiterhin wurde festgestellt, dass die unerwünschte vorzeitige Polymerisation/Gelierung unterbleibt, wenn in den eingesetzten Diisocyanatchargen ein Peroxidgehalt von kleiner als 20 mmol/kg vorliegt.

Gegenstand der vorliegenden Erfindung ist somit ein Verfahren zur Herstellung mindestens einer ethylenisch ungesättigten Urethangruppen-haltigen Verbindung aus i) Diisocyanat, ii) mindestens einer Verbindung mit mindestens einer Hydroxygruppe und mindestens einer ethylenisch ungesättigten, polymerisierbaren Gruppe und iii) optional mindestens einer hydroxygruppenhaltigen Verbindung, bei dem
1. Diisocyanat unmittelbar vor der Reaktion einer Peroxidgehaltbestimmung unterzogen wird,
2.
   a) im Falle, dass der ermittelte Peroxidgehalt größer oder gleich 20 mmol/kg beträgt, das Diisocyanat einer destillativen Aufreinigung unterzogen wird, bis der ermittelte Peroxidgehalt kleiner als 20 mmol/kg ist, oder
   b) im Falle, dass der ermittelte Peroxidgehalt kleiner 20 mmol/kg beträgt, keine destillative Aufreinigung erfolgt, und
3. anschließend i) destilliertes Diisocyanat zu a) und/oder unbehandeltes Diisocyanat zu b) mit ii) mindestens einer Verbindung mit mindestens einer Hydroxygruppe und mindestens einer ethylenisch ungesättigten, polymerisierbaren Gruppe und optional ii) mindestens einer hydroxygruppenhaltigen Verbindung zu einer ethylenisch ungesättigten Urethangruppen-haltigen Verbindung umgesetzt wird.

Gegenstand der vorliegenden Erfindung ist ein Verfahren zur Herstellung mindestens einer ethylenisch ungesättigten Urethangruppen-haltigen Verbindung aus Diisocyanat und mindestens einer Verbindung mit mindestens einer Hydroxygruppe und mindestens einer ethylenisch ungesättigten, polymerisierbaren Verbindung. Bei dem Edukt "Diisocyanat" kann es sich um ein einzelnes Diisocyanat oder ein Gemisch von Diisocyanaten handeln. Bevorzugt handelt es sich bei dem Edukt um genau ein Diisocyanat.

Bevorzugt handelt es sich bei mindestens einem der eingesetzten Diisocyanate um ein (cyclo)aliphatisches Diisocyanat, d. h. um ein Diisocyanat mit mindestens einer direkt an einen aliphatischen Ring gebundenen Isocyanatgruppe und ggf. einer weiteren aliphatisch gebundenen (d. h. an einem über einen Alkylenrest mit dem aliphatischen Ring verbundenen) Isocyanatgruppe. Weiter bevorzugt wird nur ein (cyclo)aliphatisches Diisocyanat eingesetzt. Ganz besonders bevorzugt handelt es sich bei mindestens einem der eingesetzten Diisocyanate um Isophorondiisocyanat (IPDI) oder 4,4'-Diisocyanatodicyclohexylmethan (H12MDI). Noch weiter bevorzugt wird Isophorondiisocyanat als einziges Diisocyanat eingesetzt. Wird Isophorondiisocyanat eingesetzt, ist unerheblich, ob es über das Harnstoffverfahren oder über das Phosgenverfahren erhalten wurde.

Die Verbindung mit mindestens einer Hydroxygruppe und mindestens einer ethylenisch ungesättigten, polymerisierbaren Gruppe kann prinzipiell jede beliebige Verbindung mit mindestens einer Hydroxygruppe und mindestens einer ethylenisch ungesättigten, polymerisierbaren Gruppe sein. Bevorzugt weist sie genau eine Hydroxygruppe und genau eine ethylenisch ungesättigte Gruppe auf.

Bevorzugte Verbindungen mit mindestens einer Hydroxygruppe und mindestens einer ethylenisch ungesättigten, polymerisierbaren Gruppe können ausgewählt werden aus der Gruppe bestehend aus Hydroxyalkylacrylaten, Hydroxyalkylmethacrylaten und Hydroxyalkylvinylethern. Bevorzugt handelt es sich dabei um Verbindungen, deren Hydroxyalkylgruppen C₂-C₁₀-Alkylenreste, bevorzugt lineare C₂-C₁₀-Alkylenreste aufweisen. Ganz besonders bevorzugt sind Hydroxyethylacrylat, Hydroxyethylmethacrylat, Hydroxypropylacrylat und Hydroxypropylmethacrylat,

Vor der partiellen oder vollständigen Umsetzung von Diisocyanat mit der mindestens eine Verbindung mit mindestens einer Hydroxygruppe und mindestens einer ethylenisch ungesättigten, polymerisierbaren Gruppe erfolgt eine Bestimmung des Peroxidgehaltes im einzusetzenden Diisocyanat. Der Peroxidgehalt wird bestimmt gemäß DIN EN ISO 27107 und wird bestimmt in mmol/kg.

Unter "vor" der Umsetzung ist dabei bevorzugt ein Zeitfenster von 14 Tagen bis 5 Minuten vor der Vermischung von Diisocyanat und mindestens einem Edukt zu verstehen. Ganz besonders bevorzugt ist darunter ein Zeitpunkt von 2 Tagen (48 h) vor der Umsetzung zu verstehen.

Wird nur ein Diisocyanat eingesetzt, ist der Peroxidgehalt des einzusetzenden Diisocyanats der Gehalt an Peroxid in mmol bezogen auf die Gesamtmasse des einzusetzenden Diisocyanats in Kilogramm. Wird mehr als ein Diisocyanat eingesetzt, ist der Peroxidgehalt des einzusetzenden Diisocyanats der Gesamtgehalt an Peroxid in mmol bezogen auf die Gesamtmasse aller einzusetzenden Diisocyanate in Kilogramm.

In dem Fall, dass der wie zuvor definiert ermittelte Peroxidgehalt kleiner als 20 mmol/kg ist, erfolgt keine weitere Aktion, da nennenswerte Nachteile durch die Anwesenheit von einer solchen Konzentration an Peroxid nicht zu erwarten sind. Die Edukte können somit direkt umgesetzt werden.

Ist der wie zuvor definiert ermittelte Peroxidgehalt jedoch größer oder gleich 20 mmol/kg, wird das Edukt Diisocyanat einer destillativen Aufreinigung unterzogen. Wird nur ein Diisocyanat eingesetzt, wird jede Charge des Diisocyanats, deren Peroxidgehalt größer oder gleich 20 mmol/kg ist, destillativ gereinigt, bis der Peroxidgehalt jeder Charge kleiner 20 mmol/kg beträgt. Sollen mehrere Diisocyanate eingesetzt werden, wird jede Charge jedes Diisocyanats, deren Peroxidgehalt größer oder gleich 20 mmol/kg ist, destillativ gereinigt, bis der Peroxidgehalt jeder Charge jedes Diisocyanates kleiner 20 mmol/kg beträgt.

Bevorzugt wird die destillative Aufreinigung in geeigneten Destillationskolonnen oder Destillationsaggregaten, z. B. Kurzweg- oder Dünnschichtdestillationsapparaturen, bei geeigneten Drücken und Temperaturen in Abhängigkeit der Siedetemperatur der Diisocyanate durchgeführt. Die Mindestdestillationstemperatur sollte dabei bevorzugt 100 °C nicht unterschreiten.

Neben Diisocyanat i) und mindestens einer Verbindung mit mindestens einer Hydroxygruppe und mindestens einer ethylenisch ungesättigten, polymerisierbaren Gruppe ii) kann bei der Herstellung der mindestens einen ethylenisch ungesättigten Verbindung optional weiterhin iii) mindestens eine hydroxygruppenhaltige Verbindung eingesetzt werden. Ist dies der Fall, können ethylenisch ungesättigte Urethangruppen-haltige Verbindungen mit vorteilhaften Eigenschaften hergestellt werden. Die Verbindung iii) weist keine ethylenisch ungesättigte Gruppen auf.

Bevorzugte hydroxygruppenhaltige Verbindungen iii) können ausgewählt werden aus der Gruppe bestehend aus Diolen und Polyolen. Dabei sind unter Polyolen hydroxygruppenhaltige Verbindungen mit (gemittelt) mehr als zwei Hydroxygruppen zu verstehen. Die Diole bzw. Polyole können monomer, oligomer oder polymer vorliegen. Bevorzugte oligomere und polymere Diole und Polyole weisen eine OH-Zahl von 5 bis 500 mg KOH auf (bestimmt nach DIN EN ISO 4692-2). Bevorzugte Diole und Polyole sind aliphatische Diole und Polyole, Polyesterdiole und -polyole, und Polyetherdiole und -polyole.

Bevorzugt werden Diole als hydroxygruppenhaltige Verbindungen iii) eingesetzt.

Die Umsetzung von i) Diisocyanat, ii) Verbindung mit mindestens einer Hydroxygruppe und mindestens einer ethylenisch ungesättigten, polymerisierbaren Gruppe und optional iii) hydroxygruppenhaltiger Verbindung wird bevorzugt in Gegenwart mindestens eines Katalysators bei Temperaturen von 0 - 160 °C durchgeführt. Der Druck wird dabei nicht gesondert eingestellt und entspricht dem Umgebungsdruck, der nahe bei 1 bar liegt. Bevorzugte Reaktionstemperaturen betragen 40 - 140 °C und noch weiter bevorzugt 60 - 90 °C.

Bevorzugte Reaktionszeiten liegen zwischen 3 Minuten und drei Stunden.

Geeignete Katalysatoren sind die in der Polyurethanchemie üblichen Katalysatoren. Bevorzugte Katalysatoren können ausgewählt werden aus der Gruppe bestehend aus aminischen Katalysatoren (bevorzugt Diazabicyclooctan) und Übergangsmetall-basierten Katalysatoren. Besonders bevorzugte Übergangsmetall-basierte Katalysatoren sind Dibutylzinnlaurat, Bismutneodecanoat, Zinkoctoat und Zirkoniumacetylacetonat.

Bevorzugt wird der Katalysator eingesetzt in Mengen von 0,001 - 1,5 Gew.-%, weiter bevorzugt in Mengen von 0,01 - 0,8 Gew.-%, noch weiter bevorzugt 0,05 - 0,1 Gew.-%, bezogen auf die Masse der eingesetzten Komponenten.

Obwohl prinzipiell auch Lösemittel (insbesondere Aceton, Ethylacetat, Butylacetat und Methoxypropylacetat) eingesetzt werden können, wird bevorzugt lösemittelfrei gearbeitet.

Das erfindungsgemäße Verfahren kann sowohl batchweise als auch kontinuierlich durchgeführt werden. Bevorzugt wird es im Batchverfahren durchgeführt.

Wird mindestens ein Diisocyanat i), mindestens eine Verbindung mit mindestens einer Hydroxygruppe und mindestens einer ethylenisch ungesättigten, polymerisierbaren Gruppe ii) und mindestens eine hydroxygruppenhaltige Verbindung iii) bei dem erfindungsgemäßen Verfahren eingesetzt, können prinzipiell alle Edukte i), ii) und iii) gleichzeitig miteinander umgesetzt werden. Ganz besonders vorteilhaft ist es jedoch, zuerst Diisocyanat i) mit mindestens einer Verbindung mit mindestens einer Hydroxygruppe und mindestens einer ethylenisch ungesättigten, polymerisierbaren Gruppe ii) umzusetzen und erst danach mindestens eine hydroxygruppenhaltige Verbindung iii) zuzugeben.

Aus anderen Gründen kann es jedoch auch vorteilhaft sein, zunächst Diisocyanat i) mit mindestens einer hydroxygruppenhaltigen Verbindung iii) umzusetzen und erst danach mindestens eine Verbindung mit mindestens einer Hydroxygruppe und mindestens einer ethylenisch ungesättigten, polymerisierbaren Gruppe ii) zuzugeben (Prepolymerfahrweise).

Besonders gute ethylenisch ungesättigte Urethangruppen-haltige Verbindungen resultieren weiterhin, wenn bereits im Fall, dass der ermittelte Peroxidgehalt größer oder gleich 10 mmol/kg beträgt, das Diisocyanat einer destillativen Aufreinigung unterzogen wird, bis der ermittelte Peroxidgehalt kleiner als 10 mmol/kg ist. Weiter bevorzugt wird bereits in dem Fall, dass der ermittelte Peroxidgehalt größer oder gleich 1 mmol/kg beträgt, das Diisocyanat einer destillativen Aufreinigung unterzogen, bis der ermittelte Peroxidgehalt kleiner als 1 mmol/kg ist.

Vorteilhaft ist es weiterhin, wenn das oder die Diisocyanate mindestens 0,1 mmol/kg Peroxid aufweist, d.h. jede Charge weist mindestens 0,1 mmol/kg Peroxid auf.

Weiter bevorzugt wird das erfindungsgemäße Verfahren weiterhin so durchgeführt, dass in Schritt 2.
a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg, aber größer oder gleich 0,1 mmol/kg ist,
   oder im Fall, dass der ermittelte Peroxidgehalt kleiner als 0,1 mmol/kg ist, Peroxid zugesetzt wird, bis der Peroxidgehalt größer oder gleich als 0,1 mmol/kg, aber kleiner oder gleich 10 mmol/kg ist oder
b) im Falle, dass der ermittelte Peroxidgehalt kleiner oder gleich 10 mmol/kg, aber größer oder gleich 0,1 mmol/kg ist, keine weitere Aktion erfolgt.

### Beispiele:

### Erfindungsgemäßes Beispiel

139 g Hydroxyethylacrylat (1,2 Mol, Aldrich) werden innerhalb von 30 min in eine Mischung aus 222 g Isophorondiisocyanat mit einem Peroxidgehalt < 10 mmol/kg (IPDI, 1 Mol, Evonik Industries) und 0,7g Dibutylzinndilaurat getropft und danach noch 1 h auf 70 °C erhitzt, um die Reaktion zu vervollständigen. Danach werden bei dieser Temperatur 390 g Poly THF 1000 (Du Pont, Polyetherdiol, OHZ 115) innerhalb von 30 min zugetropft und danach wieder 1 h bei 70 °C gehalten. Danach war die NCO-Zahl < 0,1%. Das Reaktionsprodukt (Urethanacrylat) ist flüssig und farblos.

### Vergleichsbeispiel

139 g Hydroxyethylacrylat (1,2 Mol, Aldrich) werden innerhalb von 30 min in eine Mischung aus 222 g Isophorondiisocyanat mit einem Peroxidgehalt von 110 mmol/kg (IPDI, 1 Mol, Evonik Industries, in das drei Tage lang bei 80 °C trockene Luft eingeblasen wurde) und 0,7g Dibutylzinndilaurat getropft und danach noch 1 h auf 70 °C erhitzt, um die Reaktion zu vervollständigen. Danach werden bei dieser Temperatur 390 g Poly THF 1000 (Du Pont, Polyetherdiol, OHZ 115) innerhalb von 30 min zugetropft und danach wieder 1 h bei 70 °C gehalten. Danach war die NCO-Zahl <0,1%. Das Reaktionsprodukt (Urethanacrylat) ist flüssig und farblos.

Beide Reaktionsprodukte wurden unter Lichtausschluss bei 60 °C gelagert. Das nach dem erfindungsmäßen Verfahren hergestellte Urethanacrylat war nach 2 Wochen bei 60 °C noch immer flüssig, ohne eine wesentliche Änderung in der Viskosität. Das Urethanacrylat basierend auf IPDI mit einem hohen Peroxidgehalt war nach 5 h bei 60 °C fest und somit nicht lagerstabil.

## Patentansprüche

1. Verfahren zur Herstellung mindestens einer ethylenisch ungesättigten Urethangruppen-haltigen Verbindung aus
i) Diisocyanat,
ii) mindestens einer Verbindung mit mindestens einer Hydroxygruppe und mindestens einer ethylenisch ungesättigten, polymerisierbaren Gruppe
iii) und optional mindestens einer hydroxygruppenhaltigen Verbindung,
bei dem
1. Diisocyanat unmittelbar vor der Reaktion einer Peroxidgehaltbestimmung unterzogen wird,
2.
a) im Falle, dass der ermittelte Peroxidgehalt größer oder gleich 20 mmol/kg beträgt, das Diisocyanat einer destillativen Aufreinigung unterzogen wird, bis der ermittelte Peroxidgehalt kleiner als 20 mmol/kg ist,
oder
b) im Falle, dass der ermittelte Peroxidgehalt kleiner 20 mmol/kg beträgt, keine destillative Aufreinigung erfolgt, und
3. anschließend i) destilliertes Diisocyanat zu a) und/oder unbehandeltes Diisocyanat zu b) mit ii) mindestens einer Verbindung mit mindestens einer Hydroxygruppe und mindestens einer ethylenisch ungesättigten, polymerisierbaren Gruppe und optional ii) mindestens einer hydroxygruppenhaltigen Verbindung zu einer ethylenisch ungesättigten Urethangruppen-haltigen Verbindung umgesetzt wird.

2. Verfahren nach Anspruch 1,
**dadurch gekennzeichnet, dass**
das Verfahren ein Diisocyanat einsetzt.

3. Verfahren nach Anspruch 1 oder 2,
**dadurch gekennzeichnet, dass**
mindestens eines der eingesetzten Diisocyanate ein (cyclo)aliphatisches Diisocyanat ist.

4. Verfahren nach einem der vorhergehenden Ansprüche,
**dadurch gekennzeichnet, dass**
mindestens eines der eingesetzten Diisocyanate Isophorondiisocyanat oder 4,4'-Diisocyanatodicyclohexylmethan ist.

5. Verfahren nach einem der vorhergehenden Ansprüche,
**dadurch gekennzeichnet, dass**
die Verbindung mit mindestens einer Hydroxygruppe und mindestens einer ethylenisch ungesättigten Gruppe ausgewählt wird aus der Gruppe bestehend aus Hydroxyalkylacrylaten, Hydroxyalkylmethacrylaten und Hydroxyalkylvinylethern.

6. Verfahren nach Anspruch 5,
**dadurch gekennzeichnet, dass**
die Alkylenreste der Hydroxyalkylacrylate, Hydroxyalkylmethacrylate oder Hydroxyalkylvinylether C₂-C₁₀-Alkylenreste sind.

7. Verfahren nach einem der vorhergehenden Ansprüche,
**dadurch gekennzeichnet, dass**
bei dem Verfahren mindestens eine hydroxygruppenhaltigen Verbindung iii) eingesetzt wird.

8. Verfahren nach Anspruch 7,
**dadurch gekennzeichnet, dass**
die mindestens eine hydroxygruppenhaltige Verbindung iii) ausgewählt ist aus der Gruppe bestehend aus aliphatischen Diolen, aliphatischen Polyolen, Polyesterdiolen und Polyesterpolyolen, und Polyetherdiolen und Polyetherpolyolen.

9. Verfahren nach einem der vorhergehenden Ansprüche,
**dadurch gekennzeichnet, dass**
die Umsetzung in Gegenwart mindestens eines Katalysators bei Temperaturen von 0 - 160 °C durchgeführt wird.

10. Verfahren nach Anspruch 9,
**dadurch gekennzeichnet, dass**
der Katalysator ausgewählt wird aus der Gruppe bestehend aus Diazabicyclooctan, Dibutylzinnlaurat, Bismutneodecanoat, Zinkoctoat und Zirkoniumacetylacetonat.

11. Verfahren nach einem der Ansprüche 7 - 10,
**dadurch gekennzeichnet, dass**
die Verfahrensführung so erfolgt, dass zuerst Diisocyanat i) mit mindestens einer Verbindung mit mindestens einer Hydroxygruppe und mindestens einer ethylenisch ungesättigten, polymerisierbaren Gruppe ii) umgesetzt wird und erst danach mindestens eine hydroxygruppenhaltige Verbindung iii) zugegeben wird.

12. Verfahren nach einem der vorhergehenden Ansprüche,
**dadurch gekennzeichnet, dass**
in Schritt 2.
a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg, aber größer oder gleich 0,1 mmol/kg ist,
oder
im Fall, dass der ermittelte Peroxidgehalt kleiner als 0,1 mmol/kg ist, Peroxid zugesetzt wird, bis der Peroxidgehalt größer oder gleich als 0,1 mmol/kg, aber kleiner oder gleich 10 mmol/kg ist oder
b) im Falle, dass der ermittelte Peroxidgehalt kleiner oder gleich 10 mmol/kg, aber größer oder gleich 0,1 mmol/kg ist, keine weitere Aktion erfolgt.
